(19) Europäisches Patentamt
European Patent Office
Office européen des brevets

(11) **EP 4 218 850 B1**

(12) **EUROPÄISCHE PATENTSCHRIFT**

(45) Veröffentlichungstag und Bekanntmachung des Hinweises auf die Patenterteilung:
**03.06.2026   Patentblatt 2026/23**

(21) Anmeldenummer: **23154169.9**

(22) Anmeldetag: **31.01.2023**

(51) Internationale Patentklassifikation (IPC):
***A61M 1/36*** (2006.01)

(52) Gemeinsame Patentklassifikation (CPC):
**A61M 1/3656;** A61M 2205/15; A61M 2205/18; A61M 2205/505

(54) **RESTLESS BUTTON ZUGANGSHÄMORRHAGIEN**

RESTLESS BUTTON ACCESS HEMORRHAGE

GESTION D'ACCES AVEC BUTTON D'AGITATION

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **01.02.2022   DE 102022102274**

(43) Veröffentlichungstag der Anmeldung:
**02.08.2023   Patentblatt 2023/31**

(73) Patentinhaber: **B. Braun Avitum AG**
**34212 Melsungen (DE)**

(72) Erfinder: **WAGNER, André**
**34117 Kassel (DE)**

(74) Vertreter: **Winter, Brandl - Partnerschaft mbB**
**Alois-Steinecker-Straße 22**
**85354 Freising (DE)**

(56) Entgegenhaltungen:
**US-A1- 2006 074 369     US-A1- 2018 126 062**
**US-A1- 2019 381 233**

**Beschreibung**

Technisches Gebiet

Die vorliegende Offenbarung betrifft eine Überwachungsvorrichtung einer Dialysemaschine zum Erkennen einer venösen Nadeldiskonnektion mittels einer venösen Drucküberwachung basierend auf einer Grenzwerteinstellung. Ferner betrifft die vorliegende Offenbarung ein Überwachungsverfahren zum Erkennen einer venösen Nadeldiskonnektion.

Hintergrund der Erfindung

Eine venöse Nadeldiskonnektion, auch Zugangshämorrhagie genannt, kommt geschätzt in jedem Dialysezentrum weltweit mindestens einmal pro Jahr vor. Bei einer solchen Nadeldiskonnektion handelt es sich um eine Unterbrechung einer bestehenden Verbindung zwischen einem venösen Zugang und einer Infusionsleitung. Eine venöse Nadeldiskonnektion kann bei nicht entdecken binnen weniger Minuten zum Tod des Patienten führen. Das Risiko bedrohlicher Diskonnektionen wird durch einen unruhigen Patienten, die Unkenntnis über ein Druck-Monitoring einer Dialysemaschine sowie einer unzureichenden Überwachung des Patienten erhöht.

Zum Überwachen des venösen Zugangs kann ein Druck des Blutes beim Eintritt in einen Katheter oder in einen Shunt genutzt werden. Hierbei findet eine venöse Druckmessung nach dem Dialysator und vor einer letzten Klemme des Dialysegeräts statt. Steigt oder fällt der Druck außerhalb eines Grenzwertbereichs so bleibt die Blutpumpe sofort stehen und löst einen patientensicheren Zustand aus.

In den bisher bekannten Systemen kann ein unterer und ein oberer Grenzwert nur während der Behandlung individuell eingestellt werden. Eine solche individuelle Einstellung ist nur mit erhöhtem Aufwand möglich, da diese Einstellungen häufig nur in einem tieferen Menüpunkt mit einem gewissen Maschinenwissen zu finden sind, wie z.B: "Untermenü Eingabe/Limits/PV/PV/oberes Δ oder OV unteres Δ".

Eine beispielsweise asymmetrische Drucküberwachung als Voreinstellung kann nur von einem technischen Service im technischen Support und Maintenance Programm (TSM) vorgenommen werden.

Ein weiterer Nachteil ist, dass ein inkrementell sinkender venöser Druck von der Dialysemaschine oftmals nicht als venöse Nadeldiskonnektion erkannt wird. Ein ggf. ausgelöster venöser Druckalarm kann zudem durch ein einfaches Quittieren durch den Anwender behoben werden, wobei das Wiederanlaufen der Blutpumpe zu einem Anpassen des aktuell herrschenden venösen Ist-Drucks führt.

Stand der Technik

Grundsätzlich ist der Zweck einer Drucküberwachung das möglichst sichere Erkennen von verschiedenen Komplikationen, die während der extrakorporalen Blutbehandlung auftreten können. Zu den möglichen Komplikationen zählt ein fehlerhafter Gefäßzugang, der beispielsweise auf das Herausrutschen oder Ansaugen der Kanüle zurückzuführen ist. Eine Druckveränderung tritt auch bei einer Veränderung des von einer Blutpumpe geförderten Blutvolumens ein.

EP3445419 A1 betrifft ein Blutbehandlungsgerät mit einer Steuerung, mit einem Pumpenaktor zum Pumpen von Blut durch einen extrakorporalen Blutkreislauf, der eine arterielle Leitung (stromauf zu einem Dialysefilter) und eine venöse Leitung (stromab zu einem Dialysefilter) umfasst, und mit einem Drucksensor zum Erfassen des Drucks in der venösen Leitung, wobei die Steuerung eine Erkennungsfunktion zum Erkennen einer venösen Nadeltrennung aufweist, die einen anhand des Drucks in der venösen Leitung ermittelten Wert mit einem Grenzwert vergleicht, um eine venöse Nadeltrennung zu erkennen, wobei der Grenzwert, mit dem die Erkennungsfunktion den Wert vergleicht und der anhand des Drucks in der venösen Leitung ermittelt wird, variabel eingestellt werden kann und/oder von der Steuerung variabel eingestellt wird.

EP3019212 A1 betrifft ein Verfahren zur Überwachung einer extrakorporalen Blutbehandlungsvorrichtung mit einem extrakorporalen Blutkreislauf und insbesondere einer venösen Nadeldiskonnektion mittels Druckpulsmessung am extrakorporalen Blutkreislauf. Des Weiteren betrifft die EP3019212 A1 eine Vorrichtung zur Durchführung eines Überwachungsverfahrens und eine Blutbehandlungsvorrichtung, die eine entsprechende Überwachungsvorrichtung enthält.

Bei den vorstehenden erwähnten bekannten Systemen ist es vorgesehen, dass bei Nichteinhaltung von bestimmten, festen Grenzwerten, ein Alarm ausgelöst wird, um die Patientensicherheit ständig und vollständig gewährleisten zu können. Während einer Dialysebehandlung ist eine dynamische Anpassung der Grenzwerte jedoch von Vorteil, da statische Grenzwerte den Nachteil haben, in bestimmten Situationen Fehlalarme auszulösen, was sich negativ auf die Dialysebehandlung auswirken kann.

US 2018/126062 A1 beschreibt ein Überwachungssystem, welches ein Verfahren zum Erkennen einer Unterbrechung einer Fluidverbindung zwischen einem ersten fluidhaltigen System und einem zweiten fluidhaltigen System durchführt.

Kurzbeschreibung der Offenbarung

Es ist die Aufgabe der vorliegenden Offenbarung, die vorstehend benannten Nachteile des Stands der Technik zu beheben oder zumindest zu vermindern und vorzugsweise einen verbesserten Umgang mit unruhigen Patienten zu ermöglichen, weiter vorzugsweise die ggf. vorhandene Unkenntnis über das Druck-Monitoring der Dialysemaschine auszuräumen und so die ggf. unzureichende Überwachung des Patienten zu verbessern, um auf diese Weise lebensbedrohliche venöse Nadeldiskonnektionen möglichst zu vermeiden und die Gebrauchstauglichkeit der Dialysemaschine zu verbessern.

Die vorstehende Aufgabe wird gelöst durch die Merkmale des Anspruchs 1. Vorteilhafte Ausgestaltungen sind Gegenstand der Unteransprüche.

Demnach wird die vorstehende Aufgabe durch eine Überwachungsvorrichtung einer extrakorporalen Blutbehandlungsmaschine, vorzugsweise Dialysemaschine zum Erkennen einer venösen Nadeldiskonnektion mittels einer venösen Drucküberwachung basierend auf einer Grenzwerteinstellung gelöst. Die Überwachungsvorrichtung hat offenbarungsgemäß einen Button (Icon), nachfolgend auch als "Restless Button" bezeichnet, welcher dazu vorgesehen und ausgebildet ist, bei einer/dessen Betätigung die Überwachungsvorrichtung zu veranlassen, einen unteren Grenzwert und einen oberen Grenzwert in Abhängigkeit eines (gemessenen) IST-Drucks einstellen zu können bzw. einzustellen. Die Überwachungsvorrichtung ist nach Ablauf einer vorbestimmten Zeit dazu vorgesehen und ausgebildet ist, den unteren Grenzwert LCL auf einen neuen unteren Grenzwert nLCL zu erhöhen, wenn sich der gleitende Mittelwert x um mehr als einen vorbestimmten Betrag erhöht hat oder den unteren Grenzwert LCL auf einen neuen unteren Grenzwert nLCL zu verringern, wenn ein Abstand zwischen einem neuen gleitenden Mittelwert x und dem unteren Grenzwert LCL kleiner ist als ein unterer Zielgrenzwert, und ein inkrementelles Absinken des venösen Drucks zu erkennen, ein inkrementelles Absinken vorliegt, wenn der neue untere Grenzwert nLCL mehr als ein vorbestimmter Prozentsatz p% eines vorherigen gleitenden Mittelwerts x abfällt.

In anderen Worten wird durch einen solchen, vorzugsweise vorkonfigurierten, "Restless Button" eine Überwachung des Patienten, insbesondere des gelegten Zugangs während einer Dialysebehandlung verbessert. Das Betätigen des Buttons löst demnach ein vorkonfiguriertes venöses Alarmfenster sowie eine Überwachung eines inkrementell sinkenden venösen Drucks aus. Bei der inkrementellen Drucküberwachung soll das Dialysesystem/ die Überwachungsvorrichtung bei Betätigen des Buttons einen inkrementell sinkenden venösen Druck erkennen. Ein inkrementell absinkender venöser Druck kann gemäß bekanntem Stand der Technik von aktuellen Algorithmen nicht erkannt werden, wenn der untere venöse Grenzwert nach Auslösen eines venösen Druckalarms oder Neueinstellen des Blutflusses auf den aktuellen Druckverlauf angepasst wird.

Der Button ist vorzugsweise ein Touch-Icon auf einem Touch-Display, welcher durch Berühren/ Drücken betätigbar ist. Anders gesagt, ist der Button ein Bedienelement in einer graphischen Benutzeroberfläche, alternativ ausgebildet als Knopf oder Taste.

Ferner ist es bevorzugt, wenn der Button auf einem Bildschirm der Dialysemaschine angeordnet ist, um die Gebrauchstauglichkeit der Überwachungsvorrichtung zu verbessern. Vorzugsweise bleibt dieser Button auf dem Bildschirm sichtbar und betätigbar unabhängig davon, welches Untermenü auf dem Bildschirm (aktuell) angezeigt wird.

Bevorzugter Weise ist der IST-Druck ein gleitender Mittelwert in einem Bereich von vorzugsweise 48 mmHg bis 69 mmHg. Eine Auswertung von etwa 20.000 Hämodialysebehandlungen zeigt ein durchschnittliches oberes venöses Druckfenster von etwa 69 mmHg und ein unteres Druckfenster von etwa 48 mmHg. Beide durchschnittlichen Druckwerte liegen deutlich oberhalb der in einem nachfolgenden Aspekt vorgeschlagenen Druckwerte von 40 mmHg und 25 mmHg, was zu einer Erhöhung der Sensitivität der Überwachung führt und im Sinne der vorliegenden Offenbarung ist.

Es ist von Vorteil, wenn der untere Grenzwert und der obere Grenzwert zu dem IST-Druck asymmetrisch ausgebildet sind. In anderen Worten, ist es vorgesehen, dass das (auf das Drücken des Buttons automatisch ausgelöste/durchgeführte) Einstellen eines unteren und eines oberen Grenzwertes ein asymmetrisches Druckfenster ergibt. Das heißt, der untere und der obere venöse Grenzwert wird bei Betätigen des Buttons (automatisch) auf eine asymmetrische venöse Drucküberwachung fest eingestellt.

Vorteilhafterweise ist eine Differenz zwischen dem oberen Grenzwert und dem IST-Druck größer als eine Differenz zwischen dem unteren Grenzwert und dem IST-Druck. Eine asymmetrische Drucküberwachung mit einer engen Heranführung der unteren Grenze führt zu einer Verbesserung der Sensitivität gegenüber einer venösen Nadeldiskonnektion.

Es ist bevorzugt, wenn der untere Grenzwert 25 mmHg unterhalb des IST-Drucks und der obere Grenzwert 40 mmHg oberhalb des IST-Drucks einstellbar ist. In anderen Worten wird der untere und der obere venöse Grenzwert bei Betätigen des Buttons auf eine asymmetrische venöse Drucküberwachung fest auf 25 mmHg unterhalb des IST-Drucks und 40 mmHg oberhalb des IST-Drucks eingestellt. Die Auswertung von den vorstehend erwähnten ca. 20.000 Hämodialysebehandlungen in Deutschland zeigte einen Anteil von 19,9 %, die unter die untere Druckgrenze von 25 mmHg vom IST-Druck gefallen sind. Das obere Druckfenster von 40 mmHg verhindert Fehlalarme, ausgelöst von Bewegungen des Shuntarms, die den ungestörten Dialyseablauf beeinträchtigen.

Es ist von Vorteil, wenn die Überwachungsvorrichtung dazu vorgesehen und ausgebildet ist, nach Ablauf 250s bis 350s, weiter bevorzugt 300 Sekunden, den unteren Grenzwert LCL auf einen neuen Grenzwert nLCL zu erhöhen, wenn sich der gleitende Mittelwert um mehr als einen vorbestimmten Betrag, vorzugsweise mehr als 2,5 mmHg erhöht hat. Das Abwarten der vorbestimmten Zeit bringt den Vorteil der Vermeidung von Fehlalarmen mit sich, da ein Patient erfahrungsgemäß zu Beginn einer Behandlung unruhig ist, bis dieser eine angenehme Position eingenommen hat.

Es ist bevorzugt, wenn die Überwachungsvorrichtung dazu vorgesehen und ausgebildet ist, nach Ablauf einer vorbestimmten Zeit, vorzugsweise 300 Sekunden, den unteren Grenzwert LCL auf einen neuen Grenzwert nLCL zu verringern, wenn ein Abstand zwischen dem neuen gleitenden Mittelwert und dem unteren Grenzwert kleiner ist als ein unterer Zielgrenzwert.

Vorteilhafterweise ist die Überwachungsvorrichtung dazu vorgesehen und ausgebildet, den neuen unteren Grenzwert nLCL zu sichern und zu überprüfen, ob ein inkrementelles Absinken vorliegt, wobei im Falle eines inkrementellen Absinkens die Überwachungsvorrichtung dazu vorgesehen ist, einen Alarm auszugeben.

Es ist bevorzugt, wenn die Überwachungsvorrichtung dazu vorgesehen und ausgebildet ist, zu überprüfen, dass der vorherig eingestellte untere Grenzwert LCL nicht mehr als einen vorbestimmten Prozentsatz p% von dem gleitenden Mittelwert der letzten vorbestimmten Zeit, vorzugsweise 300 Sekunden, abweicht. Hierbei ist es bevorzugt, wenn der Prozentsatz p% einen Wert zwischen 1% und 30% annimmt. In anderen Worten gilt:

$$\text{Inkrementell\_ALM} = LCL < nLCL * (1+p)$$

In anderen Worten, darf der neu eingestellte venöse untere Grenzwert nLCL nicht unter dem berechneten gleitenden Mittelwert zu dem vorherig gesetzten venösen unteren Grenzwert LCL liegen, der wie folgt berechnet wird:

$$PV^{\Delta} = ((\sum_{T=1}^{300} PVmin_T + PVmin_t) * \frac{1}{T+t}) * p\%$$

Diese Überwachung soll den Patienten vor, zum Beispiel, einem unerkannten Einbluten ins Gewebe schützen, indem ein absinkender Druck erkannt wird. Ferner wird der Alarm wie folgt gehandhabt: Der Button, das heißt, das automatische Setzen eines asymmetrischen Druckfensters und der Algorithmus zur Überwachung eines inkrementell sinkenden venösen Drucks ersetzt solange nicht den implementierten Algorithmus zur Überwachung des venösen Shunt-Druckes, bis der Button von einem Anwender betätigt wurde.

Es ist von Vorteil, wenn die Überwachungsvorrichtung dazu vorgesehen und ausgebildet ist, bei Unterschreiten eines unteren Grenzwerts und bei Überschreiten eines oberen Grenzwerts einen Alarm auszugeben, wobei zumindest der Alarm beim Unterschreiten des unteren Grenzwerts mittels einer PIN-Eingabe zu quittieren ist.

Bevorzugter Weise ist die Überwachungsvorrichtung dazu vorgesehen und ausgebildet, dass nach einer vorbestimmten Anzahl an mit einer PIN-Eingabe zu quittierenden Alarmen, vorzugweise zwei Alarmen, ferner bevorzugt drei Alarmen, ein zu dem vorhergehenden Alarm unterschiedlich ausgebildeter Alarm ausgegeben wird. In anderen Worten soll beim Betätigen des Buttons ein inkrementell sinkender venöser Druck derart erkannt werden, dass nach einem zweimaligen, alternativ dreimaligen Quittieren eines venösen Druckalarms ein "roter Alarm" ausgelöst wird, bleibt die Blutpumpe gestoppt. Dieser "rote Alarm" beschreibt auf einem Bildschirm der Dialysemaschine detailliert, wie der venöse Zugang zu kontrollieren ist und kann nur mittels eines PIN-Codes freigeschaltet werden. Hierbei ist es bevorzugt, wenn den PIN-Code ausschließlich im Besitz der Oberschwester ist.

In anderen Worten, wird gemäß einem Beispiel, wenn ein erster Alarm auftritt, die Blutpumpe gestoppt. Sobald dieser erste Alarm ohne PIN-Eingabe quittiert wurde, startet die Blutpumpe wieder. Sobald einer zweiter; vorzugweise dritter Alarm auftritt, stoppt die Blutpumpe erneut und der Alarm kann lediglich mittels einer PIN-Eingabe quittiert werden. Sollte das zweite bzw. dritte Quittieren zu einem "roten Alarm" führen, bleibt die Blutpumpe gestoppt.

Es ist bevorzugt, wenn bei Absinken des venösen (Shunt-) Drucks unter den unteren Grenzwert, der Anwender eine Handlungsanweisung zum Überprüfen des Shuntarms erhält und der ausgelöste Alarm ausschließlich durch eine PIN-Eingabe quittierbar ist. In anderen Worten, um die Kritikalität des venösen Druckalarms zu verdeutlichen und dem Anwender eine Handlungsanweisung zum Überprüfen des Shuntarms zu empfehlen, kann der Alarm ausschließlich mit einem PIN-Code quittiert werden. Ein Auslösen des Alarms führt zum Abschalten der Blutseite, was einen Stopp der Blutpumpe zur Folge hat und einen Austritt des Patientenblutes in die Umgebung oder in das Gewebe verhindert.

Hierbei ist es bevorzugt, dass der Alarm ohne eine PIN-Eingabe quittierbar ist/ quittiert werden muss, wenn der obere Grenzwert überschritten wird und dass der Alarm mit einer PIN-Eingabe quittierbar ist/quittiert werden muss, wenn der untere Grenzwert unterschritten wird.

Alternativ ist es bevorzugt, dass der Alarm sowohl beim Überschreiten des oberen Grenzwerts als auch beim Unterschreiten des unteren Grenzwerts mit einer PIN-Eingabe quittierbar ist/quittiert werden muss.

Vorteilhaft ist es, wenn bei Überschreiten des oberen venösen Grenzwerts ein Alarm ausgelöst wird, der die Blutpumpe stoppt. Für diesen Alarm ist für das Quittieren keine PIN-Eingabe erforderlich. Das Überschreiten des oberen venösen Drucks ist auf eine Bewegung des Patienten zurückzuführen, insbesondere ein Abknicken des venösen Blutschlauchs. Der Alarm soll dennoch detailliert beschreiben, wie die venöse Blutschlauchleitung und der venöse Gefäßzugang zu kontrollieren ist.

Ferner betrifft die vorliegende Offenbarung eine extrakorporale Blutbehandlungsmaschine, vorzugsweise Dialysemaschine, welche durch eine Überwachungsvorrichtung gemäß einem der vorhergehenden Aspekte gekennzeichnet ist.

Ferner betrifft die vorliegende Offenbarung ein Überwachungsverfahren zum Erkennen einer venösen Nadeldiskonnektion mit einer venösen Drucküberwachung basierend auf einer Grenzwerteinstellung, mit den folgenden Schritten:

- Betätigen des Buttons zum Einstellen eines unteren venösen Grenzwerts LCL und eines oberen venösen Grenzwerts ausgehend von einem IST-Druck,
- Erkennen eines Unterschreitens des unteren venösen Grenzwerts LCL,
- nach Ablauf einer vorbestimmten Zeit, Verringern des unteren Grenzwerts LCL auf einen neuen unteren Grenzwert nLCL, wenn sich der gleitende Mittelwert x um mehr als einen vorbestimmten Betrag erhöht hat oder Erhöhen des unteren Grenzwerts LCL auf einen neuen unteren Grenzwert nLCL, wenn ein Abstand zwischen einem neuen gleitenden Mittelwert x und dem unteren Grenzwert LCL kleiner ist als ein unterer Zielgrenzwert,
- Sichern des neuen unteren Grenzwerts nLCL, und
- Überprüfen, ob ein inkrementelles Absinken vorliegt, wobei ein inkrementelles Absinken vorliegt, wenn der neue untere Grenzwert nLCL mehr als ein vorbestimmter Prozentsatz p% eines vorherigen gleitenden Mittelwerts x abfällt.

Optional kann bei Vorliegen ein Alarm ausgelöst werden.

Dabei ist es erfindungsgemäß, wenn ein inkrementelles Absinken vorliegt, wenn der neue untere Grenzwert um mehr als ein vorbestimmter Prozentsatz p% unter den vorherigen gleitenden Mittelwert abfällt.

Zusammenfassend hat die vorliegende Offenbarung den Vorteil, dass die Sensitivität gegenüber einer venösen Nadeldiskonnektion verbessert sowie das Entdecken eines inkrementell sinkenden venösen Drucks ermöglicht wird. Ferner ermöglicht die vorstehende Offenbarung eine Verbesserung des Umgangs mit unruhigen Patienten, beispielsweise aus Gründen von Demenz, Blindheit, Bewusstlosigkeit, etc. Ein weiterer Vorteil ist die nun unkomplizierte Bedienung/Einstellung der optimalen Shunt-Überwachung. Dies führt zu einer höheren Sicherheit für Nacht- und Heimdialyse-Patienten und die Anzahl von lebensbedrohlichen venösen Nadeldiskonnektionen konnte reduziert werden.

Kurzbeschreibung der Figuren

Fig. 1 ist eine Darstellung zur Veranschaulichung einer Auswertung zum Ermitteln eines gleitenden Mittelwerts des venösen Drucks.

Fig. 2 ist eine Darstellung zur Veranschaulichung eines inkrementellen Absinkens gemäß dem Stand der Technik.

Fig. 3 ist eine Darstellung zur Veranschaulichung eines inkrementellen Absinkens gemäß der vorliegenden Offenbarung.

Fig. 4 ist ein Ablaufdiagramm zur Darstellung der Verfahrensschritte gemäß dem Überwachungsverfahren gemäß der vorliegenden Offenbarung.

Fign. 5 bis 7 sind jeweils eine Darstellung zur Veranschaulichung eines Displays einer Dialysemaschine.

Fig. 8 zeigt eine beispielhafte Dialysemaschine mit einem darin integrierten Display gemäß den Fign. 5 bis 7.

Beschreibung der Ausführungsbeispiele

Nachstehend werden Ausführungsbeispiele der vorliegenden Offenbarung auf der Basis der zugehörigen Figuren beschrieben.

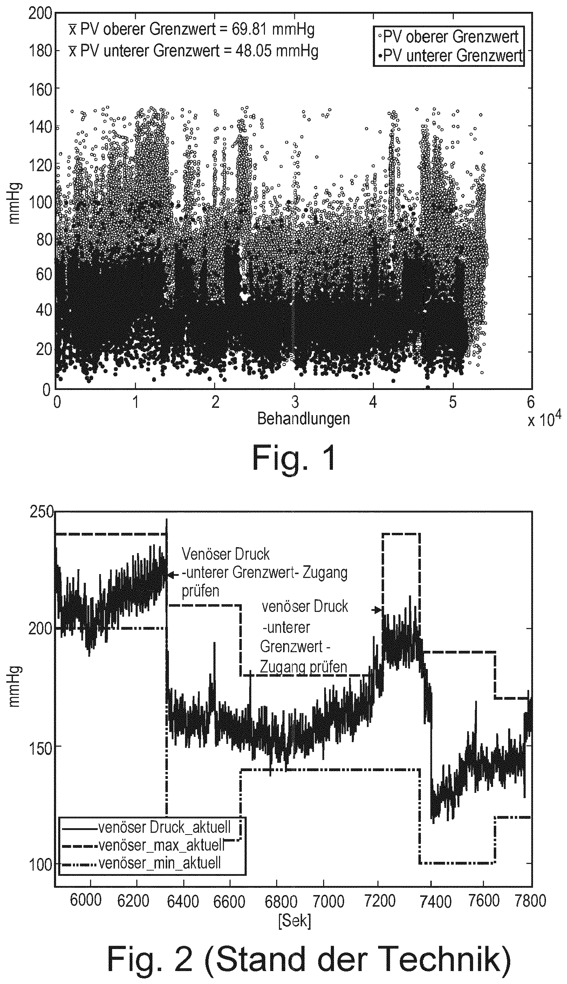

Fig. 1 ist eine Darstellung zur Veranschaulichung einer Auswertung zum Ermitteln eines gleitenden Mittelwerts des venösen Drucks. Fig. 1 ist ein Diagramm, welches auf der x-Achse die Anzahl der Behandlungen mit dem Faktor $10^4$ und auf der y-Achse den venösen Druck in mmHg anzeigt. In dem Diagramm sind leere Punkte für den venösen Druck des oberen Grenzwerts und volle Punkte für den venösen Druck (nachfolgend auch als PV bezeichnet) des unteren Grenzwerts eingezeichnet. Mittels dieser Punkte ist es möglich, einen gleitenden Mittelwert x für den oberen Grenzwert und einen gleitenden Mittelwert x für den unteren Grenzwert zu bestimmen.

Basierend auf dem Diagramm wurde eine Auswertung von etwa 20.000 Hämodialysebehandlungen durchgeführt und die Startwerte von etwa 69 mmHg als gleitender Mittelwert x für den oberen Grenzwert und von etwa 48 mmHg als gleitender Mittelwert x für den unteren Grenzwert bestimmt. Diese durchschnittlichen Druckwerte liegen deutlich oberhalb der in der vorliegenden Offenbarung verwendeten Druckwerte zum Einstellen des asymmetrischen venösen Druckfensters von 40 mmHg und 25 mmHg. Dies führt zu einer Erhöhung der Sensitivität der Überwachung.

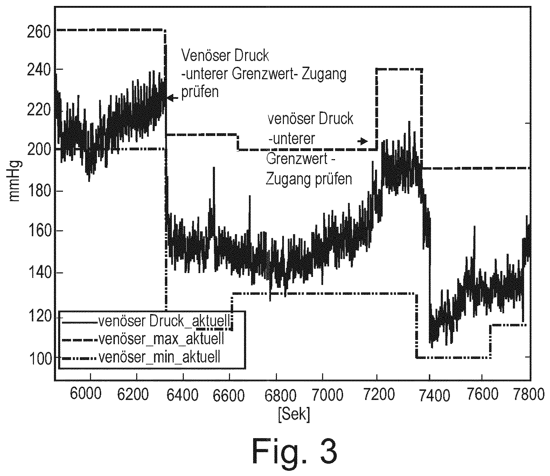

Fig. 2 ist eine Darstellung zur Veranschaulichung eines inkrementellen Absinkens gemäß dem Stand der Technik. Die Grafik der Fig. 2 sowie die Grafik der Fig. 3 hat auf der x-Achse die Zeit in Sekunden in einem Zeitbereich von 6000 Sekunden bis 7800 Sekunden und auf der y-Achse den venösen Druck in mmHg.

In der Grafik von Fig. 2 ist gemäß dem Stand der Technik ein venöser Druckverlauf gezeigt, welcher mit einer durchgehenden Linie unter der Bezeichnung "venöser Druck_aktuell" dargestellt ist. Ein erster oberer Grenzwert (nachfolgend auch als "venöser_max_aktuell" bezeichnet) ist bei 250 mmHg eingezeichnet und ein erster unterer Grenzwert (nachfolgend auch als "venöser_min_aktuell" bezeichnet) bei 200 mmHg. Nach etwa 300 Sekunden löst ein Alarm aufgrund des abgefallenen aktuellen venösen Drucks aus, wodurch der Zugang geprüft und die Grenzwerte auf den aktuell neuen Druckwert eingestellt werden. Nach weiteren 300 Sekunden wird der untere Grenzwert nochmals korrigiert, in dem Fall erhöht und der obere Grenzwert verringert. Bei Sekunde 7200 überschreiten die Druckwerte den oberen Grenzwert, wodurch lediglich der obere Grenzwert angepasst wird. Nachdem der Druckverlauf im weiteren Verlauf einen erneuten Alarm wegen eines Druckabfalls auslöst, werden die Grenzwerte gemäß dem aktuellen Druck nach unten korrigiert.

In dem Druckverlauf ist eine inkrementelle, verdeckte Druckabnahme zu erkennen. Diese venöse inkrementelle Druckabnahme kann von den bisherigen Algorithmen nicht erkannt werden, weil ein unterer Grenzwert nach Auslösen eines venösen Druckalarms oder Neueinstellen des Blutflusses, auf den aktuellen Druckverlauf angepasst wird. Hierbei wird die Art des vorherigen Alarms oder die vorherigen Alarme nicht berücksichtigt.

In der Grafik von Fig. 3, welcher dem Diagramm in Fig. 2 sehr ähnelt, ist ein venöser Druckverlauf (gemäß Fig. 2) gezeigt, welcher mit einer durchgehenden Linie unter der Bezeichnung "venöser Druck_aktuell" dargestellt ist. Ein erster oberer Grenzwert (nachfolgend auch als "venöser_max_aktuell" bezeichnet) ist bei 260 mmHg eingezeichnet und ein erster unterer Grenzwert (nachfolgend auch als "venöser_min_aktuell" bezeichnet) bei 200 mmHg. Nach etwa 300 Sekunden löst ein Alarm aufgrund des abgefallenen aktuellen venösen Drucks aus, wodurch der Zugang geprüft und die Grenzwerte auf den aktuell neuen Druckwert eingestellt werden. Nach weiteren 300 Sekunden wird der untere Grenzwert nochmals korrigiert, in dem Fall erhöht und der obere Grenzwert verringert. Bei Sekunde 7200 überschreiten die Druckwerte den oberen Grenzwert, wodurch lediglich der obere Grenzwert angepasst wird. Nachdem der Druckverlauf im weiteren Verlauf kurz vor Sekunde 7400 einen erneuten Alarm wegen eines Druckabfalls auslöst, werden die Grenzwerte gemäß dem aktuellen Druck nach unten korrigiert. Zwischen Sekunde 7600 und Sekunde 7700 wurde der untere Grenzwert erneut angepasst.

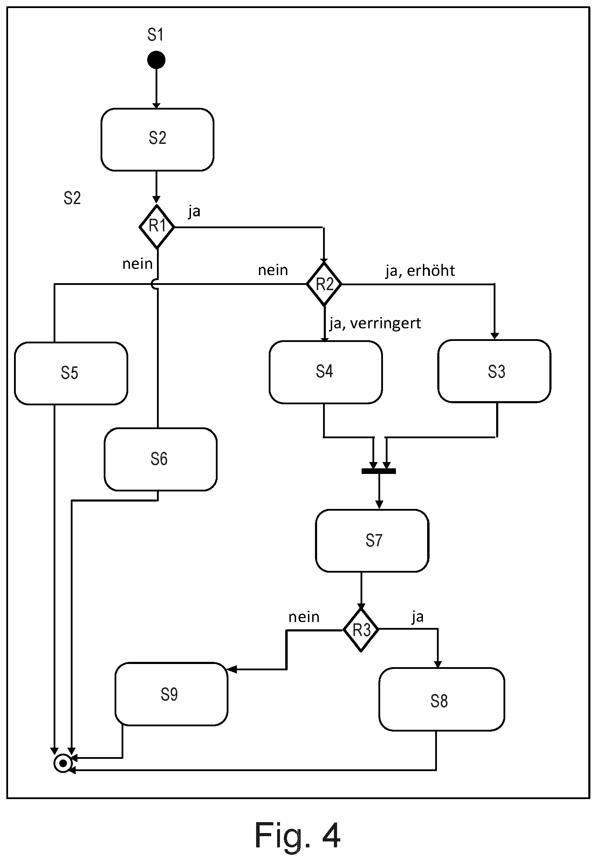

In Fig. 3 ist daher der obere und untere Grenzwert gemäß dem Druckfenster als Startwert gezeigt. Ferner zeigt Fig. 3 die gemäß der Erfindung vorgesehenen Grenzwertanpassungen, wobei das Vorgehen später zu Fig. 4 näher beschrieben wird.

Fig. 4 ist ein Ablaufdiagramm zur Darstellung der Verfahrensschritte gemäß dem Überwachungsverfahren gemäß der vorliegenden Offenbarung. In Fig. 4 wird in einem ersten Schritt S1 der Restless-Button betätigt und ausgehend von einem aktuellen venösen Druck gemäß Schritt S2 ein gleitender Mittelwert x bestimmt. Nach Betätigen des Buttons gemäß S1 wird basierend auf dem gleitenden Mittelwert x ein asymmetrisches venöses Druckfenster mit einem oberen und einem unteren Grenzwert LCL automatisch eingestellt.

In der ODER-Logik R1 wird, wenn ein Alarm ausgelöst wird, bestimmt/ geprüft, ob eine vorbestimmte Zeit von vorzugsweise 300 Sekunden seit dem letzten Einstellen des asymmetrischen Druckfensters abgelaufen ist.

Für den Fall, dass eine vorbestimmte Zeit seit dem letzten Einstellen des oberen und unteren Grenzwerts LCL gemäß R1 abgelaufen ist, also die vorbestimmte Zeit $\Delta t > 300$ Sekunden (R1: ja), wird in der ODER-Logik R2 der aktuelle venöse Druck (IST-Druck) erfasst und geprüft, ob sich der gleitende Mittelwert x um mehr als 2,5 mmHg erhöht hat. Für den Fall, dass sich der gleitende Mittelwert um mehr als 2,5 mmHg erhöht hat (R1: ja, erhöht), wird gemäß Schritt S3 der bisherige untere Grenzwert LCL entsprechend erhöht und ein neuer unterer Grenzwert nLCL ist als ein Zielgrenzwert vorgesehen.

Für den Fall, dass in R2 festgestellt wird, dass ein Abstand zwischen dem aktuellen gemittelten IST-Wert und dem bisherigen unteren Grenzwert kleiner ist als ein Zielgrenzwert, wird der untere Grenzwert gemäß Schritt S4 entsprechend verringert und ein neuer unterer Grenzwert nLCL ist als ein Zielgrenzwert vorgesehen.

Für den Fall, dass in R2 weder Schritt S3 noch Schritt S4 erfüllt ist, wird die Blutpumpe gemäß Schritt S5 (R2: nein) entsprechend dem aktuellen IST-Druck neu eingestellt.

Für den Fall, dass eine vorbestimmte Zeit seit dem letzten Einstellen des oberen und unteren Grenzwerts LCL gemäß R1 nicht abgelaufen ist, werden der untere und der obere Grenzwert LCL gemäß Schritt S6 (R1: nein) unverändert beibehalten.

Ist eine Änderung des unteren Grenzwerts in Folge auf Schritt S3 oder Schritt S4 notwendig, wird in einem Schritt S7 der neue untere Grenzwert nLCL gesichert. In einer darauffolgenden ODER-Logik R3 gilt es zu prüfen, ob der zuletzt eingestellte untere Grenzwert LCL nicht mehr als einen vorbestimmten Prozentsatz p% vom gleitenden Mittelwert x der letzten vorbestimmten Zeit, vorzugsweise der letzten 300 Sekunden, abweicht. Für den Fall, dass der bisherige untere Grenzwert LCL kleiner ist als die Summe des neuen unteren Grenzwerts nLCL und des vorbestimmten Prozentsatzes p% von dem gleitenden Mittelwert x (R3: ja), liegt gemäß Schritt S8 ein inkrementelles Absinken des venösen Drucks vor und das Gerät zeigt einen venösen Nadeldiskonnektions Alarm und die Blutpumpe stoppt. Das Gerät startet das Überwachungsverfahren erneut. Für den Fall, dass R3 nicht zutrifft, folgt das Überwachungsverfahren Schritt S9 (R3: nein) und der neue venöse untere Grenzwert nLCL wird eingestellt.

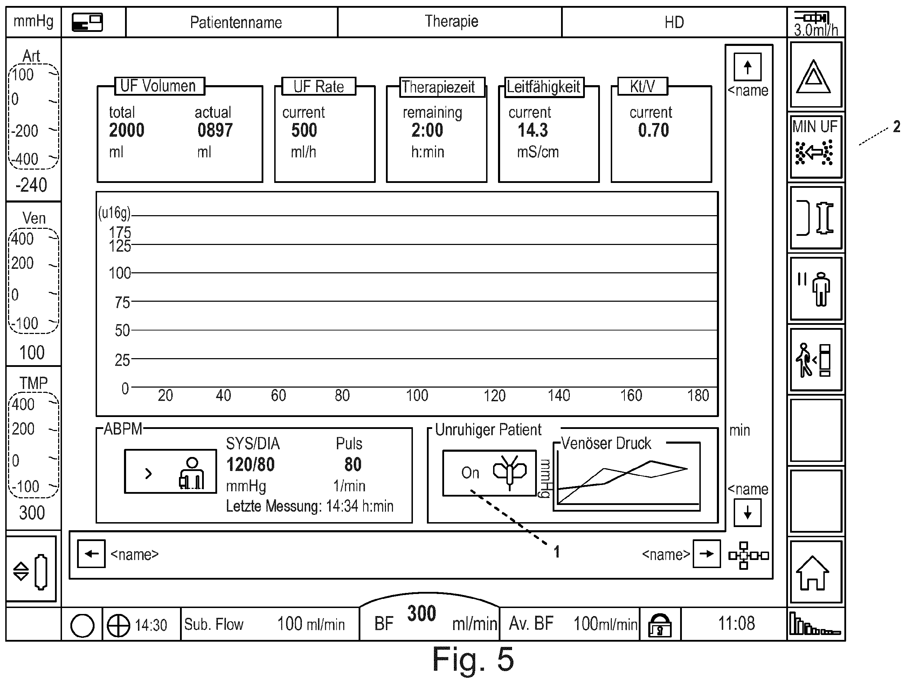

Fign. 5 bis 7 sind jeweils eine Darstellung zur Veranschaulichung eines Displays 2 einer Dialysemaschine.

Hierbei zeigt Fig. 5 die Startanzeige auf dem Display 2 mit einem Button 1 (auch "Restless Button" genannt). Der Button 1 befindet sich in einem Feld mit der Überschrift "unruhiger Patient". In diesem Feld ist ebenfalls der Druckverlauf des venösen Drucks abgebildet. Darüber hinaus sind auf dem Display 2 mit der Startanzeige sämtliche Vitalparameter des Patienten abgebildet.

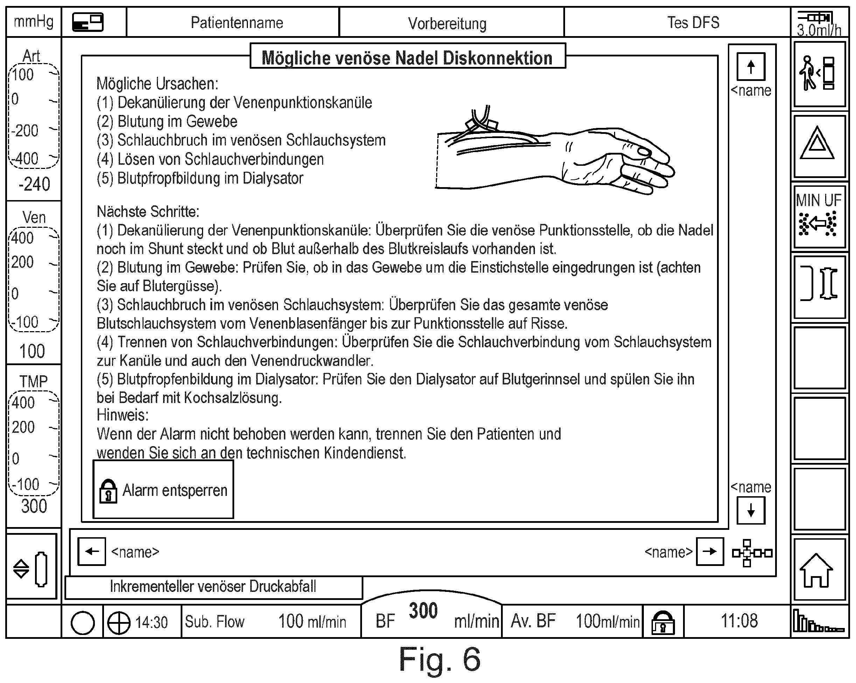

Fig. 6 zeigt, nachdem ein Alarm aufgrund einer venösen Nadeldiskonnektion ausgelöst wurde, eine detaillierte Beschreibung zur Behebung des Alarms inklusive der möglichen Ursachen für den aufgetretenen Alarm. Die nachfolgenden Punkte sind beispielhafte Ursachen für einen Alarm aufgrund des Unterschreitens eines unteren Grenzwerts:

(1) Dekanülierung der Venenpunktionskanüle
(2) Blutung im Gewebe
(3) Schlauchbruch im venösen Schlauchsystem
(4) Lösen von Schlauchverbindungen
(5) Blutpfropfbildung im Dialysator

Hierfür vorgeschlagene Lösungen sind wie folgt beispielhaft auf dem Display gezeigt:

(1) Dekanülierung der Venenpunktionskanüle: Überprüfen Sie die venöse Punktionsstelle, ob die Nadel noch im Shunt steckt und ob Blut außerhalb des Blutkreislaufs vorhanden ist.
(2) Blutung im Gewebe: Prüfen Sie, ob in das Gewebe um die Einstichstelle eingedrungen ist (achten Sie auf Blutergüsse).
(3) Schlauchbruch im venösen Schlauchsystem: Überprüfen Sie das gesamte venöse Blutschlauchsystem vom Venenblasenfänger bis zur Punktionsstelle auf Risse.
(4) Trennen von Schlauchverbindungen: Überprüfen Sie die Schlauchverbindung vom Schlauchsystem zur Kanüle und auch den Venendruckwandler.
(5) Blutpfropfbildung im Dialysator: Prüfen Sie den Dialysator auf Blutgerinnsel und spülen Sie ihn bei Bedarf mit Kochsalzlösung.

Darüber hinaus befindet sich unterhalb der detaillierten Beschreibung ein Button zum Entsperren des Alarms.

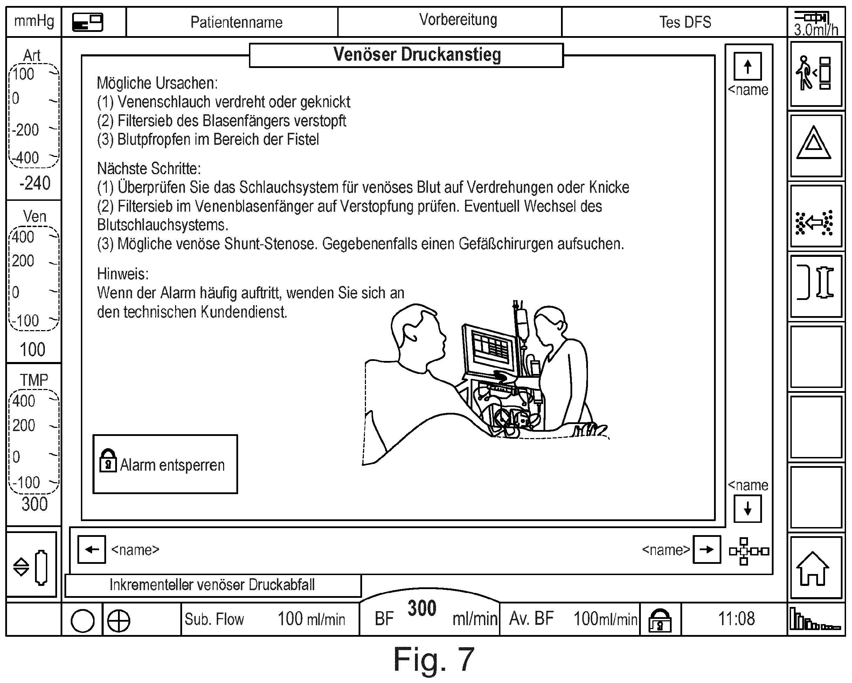

Fig. 7 zeigt, nachdem ein Alarm aufgrund eines venösen Druckanstiegs ausgelöst wurde, eine detaillierte Beschreibung zur Behebung des Alarms inklusive der möglichen Ursachen für den aufgetretenen Alarm. Die nachfolgenden Punkte sind beispielhafte Ursachen für einen Alarm aufgrund des Überschreitens eines oberen Grenzwerts:

(1) Venenschlauch verdreht oder geknickt
(2) Filtersieb des Blasenfängers verstopft
(3) Blutpfropfen im Bereich der Fistel

Hierfür vorgeschlagene Lösungen sind wie folgt beispielhaft auf dem Display gezeigt:

(1) Überprüfen Sie das Schlauchsystem für venöses Blut auf Verdrehungen oder Knicke
(2) Filtersieb im Venenblasenfänger auf Verstopfung prüfen. Eventuell Wechsel des Blutschlauchsystems.
(3) Mögliche venöse Shunt-Stenose. Gegebenenfalls einen Gefäßchirurgen aufsuchen.

Darüber hinaus befindet sich unterhalb der detaillierten Beschreibung ein Button zum Entsperren des Alarms.

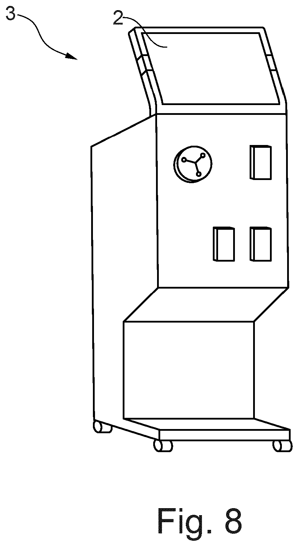

Fig. 8 zeigt eine beispielhafte Dialysemaschine 3 mit einem darin integrierten Display 2 gemäß den Fign. 5 bis 7.

**Bezugszeichen**

| | |
|---|---|
| 1 | Button |
| 2 | Display |
| 3 | Dialysemaschine |
| LCL | unterer Grenzwert |
| nLCL | neuer unterer Grenzwert |
| PV | venöser Druck |
| X | gleitender Mittelwert |
| %p | vorbestimmter Prozentsatz |

**Patentansprüche**

1. Überwachungsvorrichtung einer extrakorporalen Blutbehandlungsmaschine, vorzugsweise eine Dialysemaschine zum Erkennen einer venösen Nadeldiskonnektion mittels einer venösen Drucküberwachung basierend auf einer Grenzwerteinstellung, mit
einem Button (1), vorzugsweise angeordnet auf einem Bildschirm (2) der Dialysemaschine, welcher dazu vorgesehen und ausgebildet ist, um bei einer Betätigung die Überwachungsvorrichtung zu veranlassen, einen unteren Grenzwert (LCL) und einen oberen Grenzwert in Abhängigkeit eines IST-Drucks einzustellen, **dadurch gekennzeichnet, dass** die Überwachungsvorrichtung nach Ablauf einer vorbestimmten Zeit dazu vorgesehen und ausgebildet ist, den unteren Grenzwert (LCL) auf einen neuen unteren Grenzwert (nLCL) zu erhöhen, wenn sich der gleitende Mittelwert (x) des IST-Drucks um mehr als einen vorbestimmten Betrag erhöht hat oder den unteren Grenzwert (LCL) auf einen neuen unteren Grenzwert (nLCL) zu verringern, wenn ein Abstand zwischen einem neuen gleitenden Mittelwert (x) des IST-Drucks und dem unteren Grenzwert (LCL) kleiner ist als ein unterer Zielgrenzwert, und ein inkrementelles Absinken des venösen Drucks zu erkennen, wobei ein inkrementelles Absinken vorliegt, wenn der neue untere Grenzwert (nLCL) mehr als ein vorbestimmter Prozentsatz (p%) eines vorherigen gleitenden Mittelwerts (x) abfällt.

2. Überwachungsvorrichtung gemäß Anspruch 1, **dadurch gekennzeichnet, dass** der IST-Druck ein gleitender Mittelwert (x) in einem Bereich von vorzugsweise 48 mmHg bis 69 mmHg ist.

3. Überwachungsvorrichtung gemäß Anspruch 2, **dadurch gekennzeichnet, dass** der untere Grenzwert (LCL) und der obere Grenzwert zu dem IST-Druck asymmetrisch ausgebildet sind und/oder eine Differenz zwischen dem oberen Grenzwert und dem IST-Druck größer ist als eine Differenz zwischen dem unteren Grenzwert (LCL) und dem IST-Druck.

4. Überwachungsvorrichtung gemäß einem der Ansprüche 1 bis 3, **dadurch gekennzeichnet, dass** der untere Grenzwert (LCL) 25 mmHg unterhalb des IST-Drucks und der obere Grenzwert 40 mmHg oberhalb des IST-Drucks einstellbar ist.

5. Überwachungsvorrichtung gemäß einem der Ansprüche 1 bis 4, **dadurch gekennzeichnet, dass** die Überwachungsvorrichtung nach Ablauf der 300 Sekunden, dazu vorgesehen und ausgebildet ist, den unteren Grenzwert (LCL) auf den neuen unteren Grenzwert (nLCL) zu erhöhen, wenn sich der gleitende Mittelwert (x) um mehr als den vorbestimmten Betrag, vorzugsweise mehr als 2,5 mmHg erhöht hat oder den unteren Grenzwert (LCL) auf den neuen unteren Grenzwert (nLCL) zu verringern, wenn ein Abstand zwischen dem neuen gleitenden Mittelwert (x) und dem unteren Grenzwert (LCL) kleiner ist als den unteren Zielgrenzwert.

6. Überwachungsvorrichtung gemäß Anspruch 5, **dadurch gekennzeichnet, dass** die Überwachungsvorrichtung dazu vorgesehen und ausgebildet ist, den neuen unteren Grenzwert (nLCL) zu sichern und zu überprüfen, ob ein inkrementelles Absinken vorliegt, wobei im Falle eines inkrementellen Absinkens die Überwachungsvorrichtung dazu vorgesehen ist, einen Alarm auszugeben.

7. Überwachungsvorrichtung gemäß Anspruch 6, **dadurch gekennzeichnet, dass** die Überwachungsvorrichtung

8

dazu vorgesehen und ausgebildet ist, bei Unterschreiten eines unteren Grenzwerts (LCL) und bei Überschreiten eines oberen Grenzwerts einen Alarm auszugeben, wobei zumindest der Alarm beim Unterschreiten des unteren Grenzwerts (LCL) mittels einer PIN-Eingabe zu quittieren ist.

8. Überwachungsvorrichtung gemäß Anspruch 7, **dadurch gekennzeichnet, dass** die Überwachungsvorrichtung dazu vorgesehen und ausgebildet ist, um einer vorbestimmten Anzahl an mit einer PIN-Eingabe zu quittierenden Alarmen, vorzugweise zwei Alarmen, ferner bevorzugt drei Alarmen, ein zum ersten Alarm unterschiedlich ausgebildeter Alarm ausgegeben wird.

9. Extrakorporale Blutbehandlungsmaschine, vorzugsweise Dialysemaschine, welche durch eine Überwachungsvorrichtung gemäß einem der vorhergehenden Ansprüche gekennzeichnet ist.

10. Überwachungsverfahren zum Erkennen einer venösen Nadeldiskonnektion mit einer venösen Drucküberwachung basierend auf einer Grenzwerteinstellung, mit den folgenden Schritten:

- Betätigen des Buttons zum Einstellen eines unteren venösen Grenzwerts (LCL) und eines oberen venösen Grenzwerts ausgehend von einem IST-Druck,
- Erkennen eines Unterschreitens des unteren venösen Grenzwerts (LCL),
- nach Ablauf einer vorbestimmten Zeit, Verringern des unteren Grenzwerts (LCL) auf einen neuen unteren Grenzwert (nLCL), wenn sich der gleitende Mittelwert (x) um mehr als einen vorbestimmten Betrag erhöht hat oder Erhöhen des unteren Grenzwerts (LCL) auf einen neuen unteren Grenzwert (nLCL), wenn ein Abstand zwischen einem neuen gleitenden Mittelwert (x) und dem unteren Grenzwert (LCL) kleiner ist als ein unterer Zielgrenzwert,
- Sichern des neuen unteren Grenzwerts (nLCL), und
- Überprüfen, ob ein inkrementelles Absinken vorliegt, wobei ein inkrementelles Absinken vorliegt, wenn der neue untere Grenzwert (nLCL) mehr als ein vorbestimmter Prozentsatz (p%) eines vorherigen gleitenden Mittelwerts (x) abfällt.

**Claims**

1. A monitoring device of an extracorporeal blood treatment machine, preferably a dialysis machine for detecting a venous needle disconnection via venous pressure monitoring based on a threshold value setting, comprising

   a button (1), preferably arranged on a screen (2) of the dialysis machine, which is provided and configured, upon actuation, to cause the monitoring device to set a lower threshold value (LCL) and an upper threshold value depending on an ACTUAL pressure,
   **characterized in that** the monitoring device is provided and configured to increase the lower threshold value (LCL) to a new lower threshold value (nLCL) after a predetermined time has elapsed if the floating average value (x) of the ACTUAL pressure has increased by more than a predetermined amount, or to reduce the lower threshold value (LCL) to a new lower threshold value (nLCL) if a difference between a new floating average value (x) of the ACTUAL pressure and the lower threshold value (LCL) is smaller than a lower target threshold value, and to detect an incremental decrease in the venous pressure, wherein an incremental decrease is present if the new lower threshold value (nLCL) falls by more than a predetermined percentage (p%) of a previous floating average value (x).

2. The monitoring device according to claim 1, **characterized in that** the ACTUAL pressure is a floating average value (x) in a range of preferably 48 mmHg to 69 mmHg.

3. The monitoring device according to claim 2, **characterized in that** the lower threshold value (LCL) and the upper threshold value are asymmetrical to the ACTUAL pressure and/or a difference between the upper threshold value and the ACTUAL pressure is greater than a difference between the lower threshold value (LCL) and the ACTUAL pressure.

4. The monitoring device according to one of the claims 1 to 3, **characterized in that** the lower threshold value (LCL) is settable 25 mmHg below the ACTUAL pressure and the upper threshold value is settable 40 mmHg above the ACTUAL pressure.

5. The monitoring device according to one of claims 1 to 4, **characterized in that** the monitoring device is provided and configured to increase the lower threshold value (LCL) to the new lower threshold value (nLCL) after 300 seconds have elapsed, if the floating average value (x) has increased by more than the predetermined amount, preferably more than 2.5 mmHg, or to decrease the lower threshold value (LCL) to the new lower threshold value (nLCL) if a distance between the new floating average value (x) and the lower threshold value (LCL) is less than the lower target threshold value.

6. The monitoring device according to claim 5, **characterized in that** the monitoring device is provided and configured to save the new lower threshold value (nLCL) and to check whether an incremental decrease is present, wherein in case of an incremental decrease the monitoring device is provided to output an alarm.

7. The monitoring device according to claim 6, **characterized in that** the monitoring device is provided and configured to output an alarm when a lower threshold value (LCL) is undershot and when an upper threshold value is exceeded, wherein at least the alarm when the value falls below the lower threshold value (LCL) is to be acknowledged via a PIN entry.

8. The monitoring device according to claim 7, **characterized in that** the monitoring device is provided and configured to issue an alarm different from the first alarm for a predetermined number of alarms to be acknowledged with a PIN input, preferably two alarms, further preferably three alarms.

9. An extracorporeal blood treatment machine, preferably dialysis machine, which is **characterized by** a monitoring device according to one of the preceding claims.

10. A monitoring method for detecting a venous needle disconnection with venous pressure monitoring based on a threshold value setting, comprising the following steps:

   - actuating the button to set a lower venous threshold value (LCL) and an upper venous threshold value based on an ACTUAL pressure,
   - detecting a drop below the lower venous threshold value (LCL),
   - after a predetermined period of time has elapsed, decreasing the lower threshold value (LCL) to a new lower threshold value (nLCL) if the floating average value (x) has increased by more than a predetermined amount, or increasing the lower threshold value (LCL) to a new lower threshold value (nLCL), if the difference between a new floating average value (x) and the lower threshold value (LCL) is less than a lower target threshold value,
   - saving the new lower threshold value (nLCL), and
   - checking if an incremental decrease is present, wherein an incremental decrease is present if the new lower threshold value (nLCL) falls more than a predetermined percentage (p%) from a previous floating average value (x).

**Revendications**

1. Dispositif de surveillance d'une machine de traitement sanguin extracorporel, de préférence une machine de dialyse pour détecter une déconnexion d'aiguille veineuse au moyen d'une surveillance de pression veineuse sur la base d'un réglage de valeur limite, avec

   un bouton (1), de préférence disposé sur un écran (2) de la machine de dialyse, lequel est prévu et conçu pour, lors d'un actionnement, amener le dispositif de surveillance à régler une valeur limite inférieure (LCL) et une valeur limite supérieure en fonction d'une pression réelle, **caractérisé en ce que** le dispositif de surveillance est prévu et conçu pour, après écoulement d'un temps prédéterminé, augmenter la valeur limite inférieure (LCL) jusqu'à une nouvelle valeur limite inférieure (nLCL) si la moyenne glissante (x) de la pression réelle a augmenté de plus d'un montant prédéterminé, ou réduire la valeur limite inférieure (LCL) jusqu'à une nouvelle valeur limite inférieure (nLCL) lorsqu'un écart entre une nouvelle moyenne glissante (x) de la pression réelle et la valeur limite inférieure (LCL) est inférieur à une valeur limite cible inférieure, et détecter une baisse incrémentielle de la pression veineuse, dans lequel une baisse incrémentielle est présente lorsque la nouvelle limite inférieure (nLCL) diminue de plus d'un pourcentage prédéterminé (p%) par rapport à une moyenne glissante précédente (x).

2. Dispositif de surveillance selon la revendication 1, **caractérisé en ce que** la pression réelle est une moyenne glissante (x) dans une plage de préférence allant de 48 mmHg à 69 mmHg.

3. Dispositif de surveillance selon la revendication 2, **caractérisé en ce que** la valeur limite inférieure (LCL) et la valeur limite supérieure sont conçues de manière asymétrique par rapport à la pression réelle et/ou **en ce qu'**une différence entre la valeur limite supérieure et la pression réelle est supérieure à une différence entre la valeur limite inférieure (LCL) et la pression réelle.

4. Dispositif de surveillance selon l'une quelconque des revendications 1 à 3, **caractérisé en ce que** la valeur limite inférieure (LCL) est réglable à 25 mmHg en dessous de la pression réelle et la valeur limite supérieure à 40 mmHg au-dessus de la pression réelle.

5. Dispositif de surveillance selon l'une quelconque des revendications 1 à 4, **caractérisé en ce que** le dispositif de surveillance est prévu et conçu pour, à l'expiration de 300 secondes, augmenter la valeur limite inférieure (LCL) jusqu'à la nouvelle valeur limite inférieure (nLCL) si la moyenne glissante (x) a augmenté de plus du montant prédéterminé, de préférence de plus de 2,5 mmHg, ou réduire la valeur limite inférieure (LCL) jusqu'à la nouvelle valeur limite inférieure (nLCL) lorsqu'un écart entre la nouvelle moyenne glissante (x) et la valeur limite inférieure (LCL) est inférieur à la valeur limite cible inférieure.

6. Dispositif de surveillance selon la revendication 5, **caractérisé en ce que** le dispositif de surveillance est prévu et conçu pour enregistrer la nouvelle limite inférieure (nLCL) et vérifier si une baisse incrémentielle est présente, dans lequel le dispositif de surveillance est prévu pour émettre une alarme en cas de baisse incrémentielle.

7. Dispositif de surveillance selon la revendication 6, **caractérisé en ce que** le dispositif de surveillance est prévu et conçu pour émettre une alarme lorsqu'une valeur limite inférieure (LCL) n'est pas atteinte et lorsqu'une valeur limite supérieure est dépassée, dans lequel au moins l'alarme doit être acquittée au moyen d'une saisie de code PIN lorsque la valeur limite inférieure (LCL) n'est pas atteinte.

8. Dispositif de surveillance selon la revendication 7, **caractérisé en ce que** le dispositif de surveillance est prévu et conçu pour émettre, après un nombre prédéterminé d'alarmes devant être acquittées par saisie d'un code PIN, de préférence deux alarmes, en outre de préférence trois alarmes, une alarme conçue différemment de la première alarme.

9. Machine de traitement sanguin extracorporel, de préférence machine de dialyse, qui est **caractérisée par** un dispositif de surveillance selon l'une quelconque des revendications précédentes.

10. Procédé de surveillance pour détecter une déconnexion d'aiguille veineuse avec une surveillance de pression veineuse sur la base d'un réglage de valeurs limites, avec les étapes suivantes :

    - actionnement du bouton pour régler une valeur limite veineuse inférieure (LCL) et une valeur limite veineuse supérieure à partir d'une pression réelle,
    - détection du fait que la valeur limite veineuse inférieure (LCL) n'est pas atteinte,
    - après écoulement d'un temps prédéterminé, réduction de la valeur limite inférieure (LCL) jusqu'à une nouvelle valeur limite inférieure (nLCL) si la moyenne glissante (x) a augmenté de plus d'un montant prédéterminé, ou augmentation de la limite inférieure (LCL) jusqu'à une nouvelle limite inférieure (nLCL) si l'écart entre une nouvelle moyenne glissante (x) et la limite inférieure (LCL) est inférieur à une limite cible inférieure,
    - enregistrement de la nouvelle limite inférieure (nLCL), et
    - vérification si une baisse incrémentielle est présente, dans lequel une baisse incrémentielle est présente lorsque la nouvelle limite inférieure (nLCL) diminue de plus d'un pourcentage prédéterminé (p%) par rapport à une moyenne glissante précédente (x).

Fig. 1

Fig. 2 (Stand der Technik)

Fig. 3

Fig. 4

Fig. 5

| mmHg | | Patientenname | Vorbereitung | Tes DFS | 3.0ml/h |
|---|---|---|---|---|---|

**Mögliche venöse Nadel Diskonnektion**

Mögliche Ursachen:
(1) Dekanülierung der Venenpunktionskanüle
(2) Blutung im Gewebe
(3) Schlauchbruch im venösen Schlauchsystem
(4) Lösen von Schlauchverbindungen
(5) Blutpfropfbildung im Dialysator

Nächste Schritte:
(1) Dekanülierung der Venenpunktionskanüle: Überprüfen Sie die venöse Punktionsstelle, ob die Nadel noch im Shunt steckt und ob Blut außerhalb des Blutkreislaufs vorhanden ist.
(2) Blutung im Gewebe: Prüfen Sie, ob in das Gewebe um die Einstichstelle eingedrungen ist (achten Sie auf Blutergüsse).
(3) Schlauchbruch im venösen Schlauchsystem: Überprüfen Sie das gesamte venöse Blutschlauchsystem vom Venenblasenfänger bis zur Punktionsstelle auf Risse.
(4) Trennen von Schlauchverbindungen: Überprüfen Sie die Schlauchverbindung vom Schlauchsystem zur Kanüle und auch den Venendruckwandler.
(5) Blutpfropfenbildung im Dialysator: Prüfen Sie den Dialysator auf Blutgerinnsel und spülen Sie ihn bei Bedarf mit Kochsalzlösung.
Hinweis:
Wenn der Alarm nicht behoben werden kann, trennen Sie den Patienten und wenden Sie sich an den technischen Kindendienst.

🔒 Alarm entsperren

Art: 100 / 0 / -200 / -400 → -240

Ven: 400 / 200 / 0 / -100 → 100

TMP: 400 / 200 / 0 / -100 → 300

<name

<name

← <name>    <name> →

Inkrementeller venöser Druckabfall

○ ⊕ 14:30 | Sub. Flow 100 ml/min | BF **300** ml/min | Av. BF 100ml/min | 🔒 | 11:08

MIN UF

Fig. 6

EP 4 218 850 B1

| mmHg | ⊟ | Patientenname | Vorbereitung | Tes DFS | 3.0ml/h |

### Venöser Druckanstieg

**Art**
100
0
-200
-400
**-240**

**Ven**
400
200
0
-100
**100**

**TMP**
400
200
0
-100
**300**

Mögliche Ursachen:
(1) Venenschlauch verdreht oder geknickt
(2) Filtersieb des Blasenfängers verstopft
(3) Blutpfropfen im Bereich der Fistel

Nächste Schritte:
(1) Überprüfen Sie das Schlauchsystem für venöses Blut auf Verdrehungen oder Knicke
(2) Filtersieb im Venenblasenfänger auf Verstopfung prüfen. Eventuell Wechsel des Blutschlauchsystems.
(3) Mögliche venöse Shunt-Stenose. Gegebenenfalls einen Gefäßchirurgen aufsuchen.

Hinweis:
Wenn der Alarm häufig auftritt, wenden Sie sich an den technischen Kundendienst.

🔒 Alarm entsperren

↑ <name>

<name> ↓

← <name>

<name> →

Inkrementeller venöser Druckabfall

| Sub. Flow | 100 ml/min | BF **300** ml/min | Av. BF | 100ml/min | 🔒 | 11:08 |

## Fig. 7

EP 4 218 850 B1

Fig. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3445419 A1
- EP 3019212 A1

- US 2018126062 A1